Europäisches Patentamt

European Patent Office

Office européen des brevets

(11) Publication number: **0 127 712**
**B1**

## (12) EUROPEAN PATENT SPECIFICATION

(45) Date of publication of patent specification: **11.01.89**

(21) Application number: **83303292.3**

(22) Date of filing: **07.06.83**

(51) Int. Cl.⁴: **A 61 K 7/32,** A 61 K 39/40

(54) Deodorant containing bacterial antibodies.

(43) Date of publication of application:
**12.12.84 Bulletin 84/50**

(45) Publication of the grant of the patent:
**11.01.89 Bulletin 89/02**

(84) Designated Contracting States:
**AT BE CH DE FR GB IT LI LU NL SE**

(56) References cited:
**EP-A-0 064 103**
**GB-A-2 013 691**
**US-A-4 153 683**

(73) Proprietor: **THE STOLLE RESEARCH AND DEVELOPMENT CORPORATION**
**6990 Cornell Road**
**Cincinnati Ohio 45242 (US)**

(72) Inventor: **Beck, Lee R.**
**2550 Dunmore Place**
**Birmingham Alabama 35266 (US)**

(74) Representative: **Burford, Anthony Frederick et al**
**W.H. Beck, Greener & Co. 7 Stone Buildings Lincoln's Inn**
**London WC2A 3SZ (GB)**

Courier Press, Leamington Spa, England.

# EP 0 127 712 B1

**Description**

The present invention relates to a skin deodorant composition.

It is well known in the field of dermatology, microbiology and cosmetology that human body odor is caused by bacteria that inhabit the skin. The nutrients necessary for the growth and propagation of the bacterial are found in perspiration. It follows therefor that the greatest areas of concentration of bacterial colonies are found in areas where perspiration is the greatest which are the underarms, the groin and the like.

There are three basic ways in which a deodorant product works on the skin. In the first method a composition is applied to the skin which reduced the amount of perspiration on the skin thereby limiting the availability of nutrients for the skin bacteria. This, in turn, results in the reduction or elimination of body odor by indirectly controlling the offensive bacteria. The second general technique is cosmetic and is achieved by applying chemical agents such as fragrances to the skin which mask the odor caused by the bacteria. The third technique involves the application of a bactericidal composition to the skin which contains a chemical agent which kills the offensive bacteria. Antibacterial chemicals can be naturally or synthetically produced. Most commercial deodorant products contain perspiration regulators in combination with perfumes or fragrances. Thus, natural and synthetic compounds which exhibit bactericidal properties can be used as deordorants alone or in combination with perspiration regulators or perfumes. An example of such a bactericidal composition is disclosed in U.S. Patent 3,950,509. The disclosed compositon is based upon a protease inhibitor such as kallikrein inhibitors of the liver, lungs pancreas and the like.

It is well known to skilled immunologists that the body produces antibodies which react specifically with different species of bacteria. Moreover, the body produces different types of antibodies which function in different environments. Antibodies produced against one species of bacteria will not react with other species, and different types of antibodies have different chemical and functional properties. There are five known classes of antibodies and these classes are: IgG, IgM, IgA, IgE and IgD. Four of the antibody classes, namely IgG, IgM, IgE and IgD, are known as humoral antibodies because they naturally occur in the blood and function in those parts of the body that come in direct or indirect contact with the blood. Humoral antibodies can also be found in the tissue fluids of the body. The tissue fluids contain the humoral antibodies of the blood by diffusion of the antibodies from the blood into the surrounding tissue fluids by the process known as transudation. IgA antibody, also known as secretroy antibody, is unique in both structure and function. IgA antibody is found in the fluids secreted by the epithelial cells which line the surfaces of hollow body organs such as the respiratory tract, the cervix, the vagina, the bladder, the intestines and the like. IgA antibody functions by providing a protective barrier or film on the surface that secretes the antibody. For example, IgA antibody protects the surface of the gastrointestinal tract from infection by bacteria and viruses. IgA antibody also helps to prevent the absorption of toxins and poisons and other potentically harmful materials across the gastrointestinal tract. Similarly, IgA antibody protects the oral cavity, the respiratory tract, the cervix, the vagina, the bladder, the uterus and the inner ear from infection.

Passive immunization is the well known technique of immunizing one individual against a particular infectious disorder by administering to said individual antibodies produced in another individual. Moreover, antibodies that are found in one species of animal can be utilized in the treatment of other species. Accordingly, it is possible that antibodies produced in animal species against germs that are the cause of human body odor might be effective as a deodorant when applied topically to the skin of human beings. Thus, the concept of antibody containing deodorants, wherein the antibodies are obtained from animal species, can be deduced on the basis of contemporary knowledge in the areas of dermatology, immunology and microbiology, In this regard the milk of most species of mammals is known to contain large quantities of IgA secretory antibody. Milk, therfore, would seem to be a very logical source for deodorant antibody because it contains the same species of antibody normally found in the secretions of the animal's sebaceous glands. The milk obtained from dairy cows is an exception. The principal antibody in the milk of the cow is humoral antibody or IgG antibody. Cow's milk therefore does not contain the same type of antibody naturally found on the skin of human beings. This means that the cow is not a logical choice of species for producing antibodies of a type which occur naturally on the skin because cow's milk is a poor source of secretory antibody. Accordingly, these facts militate against the idea of using cow's milk or a product thereof as a deodorant.

An example of antibody containing milk is disclosed by *Peterson et al* in U.S. Patent 3,376,198. The anbtibody containing milk is effective in providing antibodies which counteract a number of different bacteria and viruses depending upon the antigen adminstered to a cow. *Heinbach*, U.S. Patent 3,128,230 also shows an antibody containing milk generated by administering such dead bacterial cells as *E. coli, S. viridans, D. pneumoniae* and the like to a cow. *Wilson*, U.S. Patent 3,907,987 shows an antibody containing milk which is effective against enteric disease. None of the references either show or suggest a milk product containing secretory antibodies or an antibody containing milk which is effective against skin bacteria.

In U.S. Patent No. 4324782, it is disclosed that cow's milk antibodies can be used for the prevention of specific bacterial infections in the oral cavity. For example, the antibodies present in cow's milk effective

2

EP 0 127 712 B1

against *Streptococcus mutans* can prevent dental caries when milk containing these antibodies is consumed on a daily basis because the antibody functions in the oral cavity to substantially hinder the growth and proliferation of *Streptococcus mutans* bacteria which cause dental caries. U.K. Patent Publication No. 2013691A discloses the use of cow's milk antibodies for the prevention of bacterial infections in the gastrointestinal tract. In considering these teachings, it is important to realize that it is well known to those skilled in the art of medicine that the environmental conditions in the gut and oral cavity differ dramatically. For example, the gut contains enzymes that rapidly break down proteins. Since antibodies are proteins, they are rapidly destroyed in this environment. The oral cavity, on the other hand, does not contain proteolytic enzymes. However, antibodies placed in the mouth are rapidly transported to the gut. The time of exposure of antibody in the oral cavity differs significantly to the exposure time of antibodies in the gut. Another factor is that there are differences in pH between the oral cavity, the stomach, the duodenum and colon. Since the pH differs in these organs an since pH has an effect on the function of antibodies, it stands to reason that antibody function differs in these environments. It cannot be predicted whether a specific type of antibody will check the growth and proliferation of bacteria in an anvironment where the antibody does not occur naturally. It is known that the environment of the human skin differs from that of both the oral cavity and the gastrointestinal tract. Although the sebaceous glands of the skin produce small quantities of IgA antibody, humoral antibody of the IgG type does not occur naturally on the skin. Accordingly, the status of the art is such that it is unknown whether or not IgG antibody will exhibit antibody function on the surface of the skin. A need continues to exist for an antibody containing product which is useful for the destruction of odor causing skin bacteria.

US—A—4153683 discloses that various skin diseases can be treated by topical application of a so-called "galenic immune globulin preparation". This preparation contains, as active substance, immune-globulin prepared from human blood. It is stated that the immune-globulin can be a standard gamma-globulin, a chemically stabilized or an enzymatically or hydrolytically decomposed immune-globulin. Specific reference is made to the use of non-fractionated serum which contains IgG, IgA and IgM antibodies. However, there is no suggestion that the IgG-content is effective in the treatment of skin diseases; the IgA-content being specified as "being of special interest in connection with the invention". Thus US—A—4153683 teaches only the incidental application of IgG antibodies to the skin when a non-fractionated serum is used to apply IgA antibodies.

## Disclosure of the Invention

Accordingly, one object of the present invention is to provide a topical composition comprising an antibody in a topical skin vehicle which is characterised in that the composition comprises an IgG antibody derived from the milk of a bovid animal and effective against odor-causing skin bacteria or a mixture of said IgG antibodies and said composition is a human skin deodorant.

Another object of the invention is to provide a technique of destroying odor causing skin bacteria.

Briefly, these and other objects of the present invention as hereinafter will become more readily apparent can be attained in a method of deodorizing the skin by applying to the skin a composition containing IgG antibody which is effective in destroying skin bacteria. More specifically, bovine milk antibodies including IgG antibody is an effective antibacterial agent for skin odor causing bacteria.

## Best Mode for Carrying Out the Invention

The central feature of the present invention is the finding that IgG antibody such as that found in the milk of an immunized bovid is effective as a deodorant when applied to the skin since it destroys odor causing skin bacteria. This finding is surprising because IgA type of antibodies are the only known naturally occurring skin antibodies, and because it has never been demonstrated under any conditions that IgG antibodies can function effectively in the skin environment. The present invention in its broadest aspects is the discovery that IgG antibody, no matter how it is derived, is effective against skin bacteria when the antibody is applied to the skin.

In the preferred embodiment of the invention, milk obtained from a bovid such as the cow, which has been challenged with antigen, is an excellent source of IgG antibody. Any combination of antigens which, when administered to a bovid, result in a milk product containing IgG antibody, against skin colonizing bacteria can be used. The following vaccine containing a variety of bacterial antigens which colonize skin is effective in producing a milk product containing IgG antibody when administered in cows:

(1) *Staphylococcus aureus;*
(2) *Staphylococcus epidermis;*
(3) *Streptococcus pyogenes,* Group A;
(4) *Salmonella enteritides;*
(5) *Aerobacter aerogenes;*
(6) *Pseudomonas aeruginosa;*
(7) *Escherichia coli;*
(8) *Corynebacterium diphtheriae* and acne;
(9) *Haemophilus influenzae.*

Methods of producing vaccines are well known to those skilled in the art. Moreover, the present invention is not directed to a novel vaccine, nor a method of production of a vaccine nor a method of

3

# EP 0 127 712 B1

administering a vaccine. The only important factor in producing the vaccine used from the standpoint of the invention is that the vaccine contains one or more antigenic bacterial species that colonize on the human skin. The minimum number of antigens which are needed to produce an IgG antibody containing milk that is effective in preventing body odor is one. The maximum number is equal to the bacterial species capable of inhabiting the skin and which cause body odor. This number is not a known quantity.

In the preparation of the antibody containing milk of the present invention, the bovid may be any milk producing member of genus *Bos*, preferably cows, sheep and goats, most preferably cows.

The bovid selected for the preparation of the IgG antibody containing milk is brought into a state of specific immunization by a suitable technique well known to those skilled in the art. Generally, the bovid is immunized in two stages. An example of this technique is fully described in U.S. Patent 4,284,623 and in the specific example which follows.

Another source for IgC antibody which could be used is the known monoclonal technique for producing antibodies. The first step in any cloning technique employed is the generation of appropriate immunocytes, i.e. cells capable of generating IgG antibody in response to an antigenic challenge. The immunocytes obtained are subsequently fused with an appropriate immortalizing cell line such as mouse myeloma or rabbit myeloma by the virus-induced technique, polyethylene glycol treatment or the like. Subsequent to fusion the hybrid cells are grown by various techniques such as in a selective growth medium a maintenance medium or by the use of feeder cells. The hybrid colonies obtained are screened in order to identify the presence of hybrid cells which produce the IgC antibody desired. The hybrid cells obtained must be cloned in order to isolate colonies of the hybrid cells which yield the desired IgG antibody. Once such cells are located and isolated, known methods can be employed to maximize the production of monoclonal antibodies.

The IgG antibody containing material can be applied to the skin in a variety of ways in order to accomplish the intended objective of deodorizing the skin. The antibody containing substance such as antibody milk or monoclonal antibody can be applied directly to the skin or a vehicle commonly used in the manufacture of compositions which are topically applied to the skin may be mixed with the antibody containing milk to form a composition which can be applied to the skin. Suitable such vehicles include gels, liquid sprays (pump and aerosols), creams, liquid, lotions, oils, powders, ointments, solid stick applicators and the like.

Normally, when antibody milk is used, the preparation would comprise from 2% to 25% by wt. of the milk in the cosmetic vehicle. The antibodies can be extracted from the antibody containing milk and applied directly or in a topical skin vehicle. Alternatively, a milk product such as whey containing the antibodies can be used as it is or blended with a topical skin vehicle. The concentration range of antibodies in differnt topical skin compositions varies relative to the vehicle employed. The minimum effective concentration must be established specifically for each and every formulation with the amount being that which provides an acceptable deodorizing effect.

In another embodiment of the invention, the antibody milk can be converted to powdered form by well known techniques. The powdered milk could itself be used as the deodorant or it may be mixed with one of the above-mentioned vehicles commonly used in skin treatment preparations. The powdered milk can be blended with the appropriate vehicle in an amount of 1 to 25 wt. %.

The antibody containing substance, preferably as the milk or a milk product such as powdered milk, can be incorporated in various human skin cleansing bases such as soap, both solid and liquid, with treatment of the skin occurring while one bathes. The antibody containing material can also be incorporated in other cleansers and disinfectants. In an embodiment of deodorization by cleansing, the antibody milk product or powdered milk can be added to bath water in amounts ranging from 5 to 25 weight percent. The concept can be extended even further to the cleansing of clothes by incorporating the antibody milk or powdered milk either into the wash water for the clothes or into the soap or detergent which is to be used for cleansing the clothes. Still further, the clothes being washed could be treated during the rinse cycle of the washing procedure and not just during the washing cycle.

IgG antibody suitable for use as a deodorant can be obtained as a monoclonal antibody as previously explained or it can be obtained in the form of other milk products such as whey, cream, low fat milk, skim milk and the like. Just like whole milk, these milk products and the monoclonal antibody can be directly applied to the skin or the antibody containing substance can be placed in a suitable cosmetic vehicle.

The mechanism by which the applied IgG antibodies effect the deodorization of the skin is not known. The effect of the antibodies could be bactericidal. On the other hand, it is possible that the antibodies prevent skin bacteria from adhering to the skin, but rather to the hair thereby inhibiting the growth and colonization of the bacteria.

In order to achieve effective deodorization of the skin, the IgG antibody containing substance should be applied in quantities which apply from 1 to 30 mg of antibodies per application. A more preferred range of application is 5 to 15 mg of antibody per application.

While the antibody containing substance of the present invention can be applied to any skin surface of the human body, it preferably finds utility in areas of the skin more prone to release offensive odors such as under the arms, the feet and the groin. The antibody containing substance itself may be applied to the desired area of the body or it may be applied as the active ingredient in a composition comprising the antibody containing substance and an inert vehicle. For application to the various parts of the body any

4

type of formulation containing the antibody can be applied. When applied to the feet, sprays and powders containing the antibody are probably the most preferred compositions. On the other hand, when the antibody is applied to the underarms, sprays, creams, ointments and solid stick formulations are probably the most preferred compositions. For application to the groin, the most preferred formulations for application are probably spray compositions and powders and perhaps ointments and creams. Other types of antibody containing formulations include pump sprays, solid stick applicators, gels and liquids as well as topical skin cleansers and disinfectants. In all formulations it is important that the antibodies be compatible with the ingredients of the topical skin vehicle, that is, the antibodies must maintain their viability in formulations.

Having generally described this invention, a further understanding can be obtained by reference to certain specific examples which are provided herein for purpose of illustration only and are not intended to be limiting unless otherwise specified.

Example

Preparation of the Vaccine

The following bacterial strains were obtained from the American Type Culture Collection, which ensures authenticity of bacterial strain and the highest standard of purity that is available.

(1) *Staphylococcus aureus;*
(2) *Staphylococcus epidermis;*
(3) *Streptococcus pyogenes,* Group A;
(4) *Salmonella enteritides;*
(5) *Aerobacter aerogenes;*
(6) *Pseudomonas aeruginosa;*
(7) *Escherichia coli;*
(8) *Corynebacterium diphtheriae* and acne;
(9) *Haemophilus influenzae.*

Upon receipt, each individual bacterial strain was grown on a blood agar plate to test the viability of the culture and to determine if the growth pattern is typical or atypical of the bacteria in question. A single colony from each of the test cultures was taken for histological examination to further ensure authenticity and purity of the culture. A single colony of each culture was used to inoculate 500 ml of standard culture broth. The standard broths recommended by the American Type Culture Collection were used to grow each of the specific bacteria listed.

All organisms were incubated as static cultures. Each culture was cultivated for 48 hours at 37°C. Following incubation, the cultures were killed by heating at 60°C for two hours. Samples of the killed bacteria were used to inoculate fresh broth which was then incubated for 24 hours at 37°C to determine if the killing process was complete. Only cultures proven sterile by this procedure were used for further processing. Sterile cultures were then washed five times in distilled water and the cells were recovered by centrifugation. The bacterial cells were frozen by immersion in liquid nitogen and freeze dried by the process of lyophilization. The lyophilized cells were stored in sterile vials until used for production of the polyvalent vaccine. the polyvalent vaccine was prepared by weighing out one gram quantities of each of the bacterial strains. The dry cells were mixed together and this mixture was suspended in sterile physiological saline (20 grams of bacteria per 500 ml saline).

A sample of the concentrated solution was diluted in serial fashion with saline to determine dilution which gives a concentration of $4 \times 10^8$ ml per ml. The stock concentrated polyvalent vaccine was dispersed into multiple containers and stored frozen. The final dilution of concentrate was made just prior to immunization. The preferred procedure is to remove a sufficient number of vials to immunize the number of cows to be treated. For example, the vials are removed 24 hours prior to the planned time of immunization; a sample of the concentrate is then diluted in a sterile container to a final concentration of $4 \times 10^8$ cells per ml. The maximum response in cows is obtained by injecting $20 \times 10^8$ bacterial cells or 5 ml of the sterile preparation which is $4 \times 10^8$ cells per ml according to the method of immunization described below.

Immunization of Cows

The antibody product of the invention is produced by immunizing cows with the polyvalent antigen prepared as described above. Five cows are injected with 5 cc of polyvalent antigen containing $20 \times 10^8$ bacterial cells. The injection was made intramuscularly in the gluteus maximum muscle of the hind leg. This procedure was repeated at one week intervals for four consecutive weeks beginning 2—3 weeks prior to the predicted day of parturtion. Following the primary immunization, booster injections using the same concentration of the antigen, were given every 14 days. This method of immunization gives the maximum antibody titer.

Collection, Handling and Processing of Milk

The milk was collected from immunized cows in a modern dairy parlor. A fully automated milking system collects and stores the milk under completely sanitary conditions. The milking system consists of automated machines connected directly to refrigerated storage tanks by a closed system of pipes. The complete system is cleaned and sterilized following each milking to ensure maximum sanitary conditions. It is important to take careful steps to prevent the growth of bacteria in immune milk during processing, since such bacteria can lower the titer of antibodies in the milk.

Milk from the 5 immunized cows was transported directly to the laboratory. Immunohistochemical methods which utilized column chromatography were used to separate the immunoglobulin fraction from the whole fresh milk. Briefly, the steps involved (1) separation of the fat by centrifugation (2) removal of the caesin by acid precipitation to obtain whey proteins and (3) separation of the IgG fraction from the whey protein by column chromatography. The details of these methods are well known to those skilled in the art and various modifications of these basic steps have been reported with satisfactory results. General reference for these procedures is Butler, *Journal of Dairy Science 55*, No. 2, pp. 151—164, 1972 and Newby, *Journal of Immunology, 118*, No. 2, pp. 461—465, 1977.

Clinical Test for Deodorant Activity

An antibody containing deodorant composition was prepared by mixing IgG antibody obtained as described above with talc in an amount of 1.5% antibody in the mixture. A 0.4 g sample containing either 6 or 60 mg antibody was applied to individual underarms of various human subjects.

In analyzing for the effectiveness of the antibody containing formulations, the underarm areas were sampled eight hours after application by blotting each underarm with a sterile cotton cloth for 30 seconds. Thereafter, each underarm was sampled with a sterile velvet-covered pad by pressing the pad against the armpit for 10 seconds. Each pad was pressed serially onto three agar plates, 3 sec on each plate. The agar plates were then incubated for 16 hours at 37°C, and after incubation the bacterial colonies were counted.

Controls:

For the control experiment, each underarm area in the test was sampled in the morning before application of the talc and in the afternoon, 8 hours after application of the talc based formulation. The subjects in the test had not used a deodorant composition or a deodorant-containing soap for a period of four days prior to the test. In the application of the talc composition, each underarm was blotted with a sterile cotton cloth for 30 seconds, the underarm was sampled with a velvet pad as described above, and then the underarm area was dusted with 0.4 g of talc. As described above, bacterial colony counts were obtained for the agar plate samples obtained. The results of the test are shown in the following table.

These results clearly demonstrate that the application of antibodies derived from milk obtained from cows specifically immunized against odor causing bacteria inhibits the colonization and/or growth and proliferation of bacterial species that normally inhabit the skin. Since these same bacterial species are known to cause body odor this product has application as a deodorant.

ANTIBODY DEODORANT PRELIMINARY TEST RESULTS

| Subject type | No. of subjects | Left arm[a] | Result | Right arm[a] | Result | Handness |
|---|---|---|---|---|---|---|
| Negroid, male | 1 | 688/221 | + | TNTC[b]/498 | + | R |
| Negroid, male | 1 | TNTC/68 | + | 490/380 | + | L |
| Negroid, female | 1 | 670/390 | + | 860/226 | + | R |
| Negroid, female | 1 | TNTC/TNTC | ? | TNTC/TNTC | ? | R |
| Caucasian, male | 1 | 53/21 | + | 45/165 | – | R |
| Caucasian, male | 1 | TNTC/297 | + | 610/248 | + | R |
| Caucasian, male | 1 | 63/129 | – | 700/201 | + | R |
| Caucasian, male | 3 | TNTC/TNTC | ? | TNTC/TNTC | ? | R |
| Caucasian, female | 1 | 106/TNTC | – | 145/0 | + | R |
| Caucasian, female | 1 | 333/20 | + | 204/21 | + | R |

Total armpits tested = 24

Total positive = 13    Total negative = 3    Total ambiguous = 8

[a] Results given as control/antibody.

[b] TNTC = too numerous to count (>1000).

**Claims**

1. A topical composition comprising an antibody in a topical skin vehicle, characterised in that the composition comprises an IgG antibody derived from the milk of a bovid animal and effective against odor-causing skin bacteria or a mixture of said IgG antibodies and said composition is a human skin deodorant.

2. A topical composition as claimed in Claim 1, wherein said composition is substantially free of IgA antibodies.

3. A composition as claimed in Claim 1 or Claim 2, comprising a mixture of a spectrum of IgG antibodies effective against odor-causing skin bacteria.

4. A composition as claimed in Claim 3, wherein said mixture comprises IgG antibodies effective against:—

  (1) *Staphylococcus aureus;*
  (2) *Staphylococcus epidermis;*
  (3) *Streptococcus pyogenes,* Group A;
  (4) *Salmonella enteritides;*
  (5) *Aerobacter aerogenes;*
  (6) *Pseudomonas aeruginosa;*
  (7) *Escherichia coli;*
  (8) *Corynebacterium diphtheriae* and acne; and
  (9) *Haemophilus influenzae.*

5. A composition as claimed in any one of the preceding Claims, wherein said IgG antibodies are monoclonal antibodies obtained from hybridoma cells derived from immunocytes from a bovid host immunized against odor-causing skin bacteria.

6. A composition as claimed in any one of Claims 1 to 4, wherein said IgG antibodies are provided in milk from a bovid immunized against odor-causing skin bacteria.

7. A composition as claimed in Claim 6, wherein said bovid is the dairy cow.

8. A composition as claimed in Claim 6 or Claim 7, wherein said IgG antibodies are obtained by extraction of milk containing said antibodies.

9. A composition as claimed in any one of Claims 5 to 8, wherein the bovid has been immunized using a vaccine containing:—

  (1) *Staphylococcus aureus;*
  (2) *Staphylococcus epidermis;*
  (3) *Streptococcus pyogenes,* Group A;
  (4) *Salmonella enteritides;*
  (5) *Aerobacter aerogenes;*
  (6) *Pseudomonas aeruginosa;*
  (7) *Escherichia coli;*
  (8) *Corynebacterium diphtheriae* and acne; and
  (9) *Haemophilus influenzae.*

10. A composition as claimed in any one of the preceding claims, wherein from 1 to 25 wt % of said IgG antibodies is present in said mixture.

11. A process for deodorizing skin by applying to the skin a skin deodorant, characterised in that the skin deodorant applied to the skin comprises an IgG antibody effective against odor-causing skin bacteria.

12. A process for deodorizing a situs of odor-causing skin bacteria by applying to said situs a deodorant, characterised in that said deodorant applied to the situs comprises an IgG antibody effective against odor-causing skin bacteria.

13. A process for preparing a skin deodorant composition by incorporating a skin deodorant into a topical skin vehicle, characterised in that said skin deodorant incorporated into the vehicle comprises an IgG antibody effective against odor-causing bacteria.

14. Use, except private use, of an IgG antibody effective against odor-causing skin bacteria to deodorize skin.

15. Use of an IgG antibody effective against odor-causing skin bacteria in the manufacture of a composition for deodorising skin.

**Patentansprüche:**

1. Topische Zusammensetzung, die einen Antikörper in einem topischen Hautträger umfaßt, dadurch gekennzeichnet, daß die Zusammensetzung einen IgG-Antikörper, der von der Milch eines zu den Rindern gehörenden Tieres stammt und gegen geruchsverursachende Hautbakterien wirksam ist, oder eine Mischung dieser IgG-Antikörper umfaßt, und daß die genannte Zusammensetzung ein Desodorierungsmittel für menschliche Haut ist.

2. Topische Zusammensetzung, wie in Anspruch 1 beansprucht, wobei diese Zusammensetzung im wesentlichen frei von IgA-Antikörpern ist.

3. Zusammensetzung, wie in Anspruch 1 oder 2 beansprucht, die eine Mischung eines Spektrums von IgG-Antikörpern umfaßt, die gegen geruchsverursachende Hautbakterien wirksam sind.

# EP 0 127 712 B1

4. Zusammensetzung, wie in Anspruch 3 beansprucht, worin die genannte Mischung IgG-Antikörper umfaßt, die gegen
(1) Staphylococcus aureus,
(2) Staphylococcus edidermis,
(3) Streptococcus pyogenes, Gruppe A,
(4) Salmonella enteritides,
(5) Aerobacter aerogenes,
(6) Pseudomonas aeruginosa,
(7) Escherichia coli,
(8) Corynebacterium diphtheriae und acne und
(9) Haemophilus influenzae
wirksam sind.

5. Zusammensetzung, wie in einem der vorhergehenden Ansprüche beansprucht, wobei die genannten IgG-Antikörper monoklonale Antikörper sind, die von Hybridenzellen erhalten wurden, welche von Immunozyten von einem Rinderwirt stammen, der gegen geruchsverursachende Hautbakterien immunisiert ist.

6. Zusammensetzung, wie in einem der Ansprüche 1 bis 4 beansprucht, wobei die genannten IgG-Antikörper in Milch von einem gegen geruchsverursachende Hautbakterien immunisierten Rind vorgesehen sind.

7. Zusammensetzung, wie in Anspruch 6 beansprucht, wobei dieses Rind eine Milchkuh ist.

8. Zusammensetzung, wie in Anspruch 6 oder 7 beansprucht, wobei die genannten IgG-Antikörper durch Extrahieren von die genannten Antikörper enthaltender Milch erhalten werden.

9. Zusammensetzung, wie in einem der Ansprüche 5 bis 8 beansprucht, wobei das Rind unter Verwendung einer Vakzine immunisiert wurde, die
(1) Staphylococcus aureus,
(2) Staphylococcus edidermis,
(3) Streptococcus pyogenes, Gruppe A,
(4) Salmonella enteritides,
(5) Aerobacter aerogenes,
(6) Pseudomonas aeruginosa, ·.
(7) Escherichia coli,
(8) Corynebacterium diphtheriae und acne und
(9) Haemophilus influenzae
enthält.

10. Zusammensetzung, wie in einem der vorhergehenden Ansprüche beansprucht, wobei 1 bis 25 Gew.% der genannten IgG-Antikörper in dieser Mischung vorhanden sind.

11. Verfahren zum Desoderieren von Haut durch Aufbringen eines Hautdesodorierungsmittels auf die Haut, dadurch gekennzeichnet, daß das auf die Haut aufgebrachte Desodorierungsmittel einen IgG-Antikörper aufweist, der gegen geruchsverursachende Hautbakterien wirksam ist.

12. Verfahren zum Desodorieren eines Situs von geruchsverursachenden Hautbakterien durch Aufbringen eines Desodorierungsmittels auf diesen Situs, dadurch gekennzeichnet, daß das auf den Situs aufgebrachte Desodorierungsmittel einen IgG-Antikörper aufweist, der gegen geruchsverusachende Bakterien wirksam ist.

13. Verfahren zum Herstellen einer Hautdesodorierungsmittelzusammensetzung durch Einverleiben eines Hautdesodorierungsmittels in einen topischen Hautträger, dadurch gekennzeichnet, daß dieses dem Träger einverleibte Hautdesodorierungsmittel einen IgG-Antikörper aufweist, der gegen geruchsverursachende Bakterien wirksam ist.

14. Verwendung, ausgenommen private Verwendung, eines IgG-Antikörpers, der gegen geruchsverursachende Bakterien wirksam ist, zum Desodorieren von Haut.

15. Verwendung eines IgG-Antikörpers, der gegen geruchsverursachende Hautbakterien wirksam ist, bei der Herstellung einer Zusammensetzung zum Desodorieren von Haut.

**Revendications**

1. Composition à usage local comprenant un anticorps dans un véhicule à usage dermatologique local, caractérisée en ce que la composition comprend un anticorps IgG dérivé du lait d'un bovin et efficace contre les bactéries de la peau qui sont à l'origine d'odeurs ou un mélange desdits anticorps IgG, et en ce que ladite composition est un déodorant pour la peau humaine.

2. Composition à usage local selon la revendication 1, où ladite composition est essentiellement dépourvue d'anticorps IgA.

3. Composition selon la revendication 1 ou la revendication 2, comprenant un mélange d'un spectre d'anticorps IgG efficaces contre les bactéries de la peau qui sont à l'origine d'odeurs.

8

4. Composition selon la revendication 3, où ledit mélange comprend des anticorps IgG efficaces contre:

(1) *Staphylococcus aureus*;
(2) *Staphylococcus epidermis*;
(3) *Streptococcus pyogenes*, Groupe A;
(4) *Salmonella enteritides*;
(5) *Aerobacter aerogenes*;
(6) *Pseudomonas aeruginosa*;
(7) *Escherichia coli*;
(8) *Corynebacterium diphtheriae* et *acnes*; et
(9) *Haemophilus influenzae*.

5. Composition selon l'une quelconque des revendications précédentes, où lesdits anticorps IgG sont des anticorps monoclonaux obtenus à partir de cellules d'hybridomes dérivées d'immunocytes d'un hôte bovidé immu8nisé contre les bactéries de la peau qui sont à l'origine d'odeurs.

6. Composition selon l'une quelconque des revendications 1 à 4, où lesdits anticorps IgG sont fournis dans le lait provenant d'un bovidé immunisé contre les bactéries de la peau qui sont à l'origine d'odeurs.

7. Composition selon la revendication 6, où ledit bovidé est la vache laitière.

8. Composition selon la revendication 6 ou la revendication 7, où lesdits anticorps IgG sont obtenus par extraction de lait contenant lesdits anticorps.

9. Composition selon l'une quelconque des revendication 5 à 8, où le bovidé a été immunisé en utilisant un vaccin contenant:

(1) *Staphylococcus aureus*;
(2) *Staphylococcus epidermis*;
(3) *Streptococcus pyogenes*, Groupe A;
(4) *Salmonella enteritides*;
(5) *Aerobacter aerogenes*;
(6) *Pseudomonas aeruginosa*;
(7) *Escherichia coli*;
(8) *Corynebacterium diphtheriae* et *acnes*; et
(9) *Haemophilus influenzae*.

10. Composition selon l'une quelconque des revendications précédentes, où de 1 à 25% en poids desdits anticorps IgG sont présents dans ledit mélange.

11. Procédé pour désodoriser la peau en appliquant à la peau un désodorant cutané, caractérisé en ce que le déodorant cutané appliqué à la peau comprend un anticorps IgG efficace contre les bactéries de la peau qui sont à l'origine d'odeurs.

12. Procédé pour désodoriser un lieu d'implantation de bactéries de la peau qui sont à l'origine d'odeurs en appliquant audit lieu un déodorant, caractérisé en ce que ledit déodorant appliqué à ce lieu comprend un anticorps IgC efficace contre les bactéries de la peau qui sont à l'origine d'odeurs.

13. Procédé de préparation d'une composition de déodorant cutané par incorporation d'un déodorant cutané à un véhicule cutané à usage local, caractérisé en ce que ledit déodorant cutané incorporé dans le véhicule comprend un anticorps IgG efficace contre les bactéries qui sont à l'origine d'odeurs.

14. Application, en-dehors de l'utilisation privée, d'un anticorps IgG efficace contre les bactéries de la peau qui sont à l'origine d'odeurs pour désodoriser la peau.

15. Application d'un anticorps IgG efficace contre les bactéries de la peau qui sont à l'origine d'odeurs à la préparation d'une composition pour désodoriser la peau.